(19) Europäisches Patentamt
European Patent Office
Office européen des brevets

(11) EP 4 667 479 A1

(12) **EUROPEAN PATENT APPLICATION**
published in accordance with Art. 153(4) EPC

(43) Date of publication:
**24.12.2025 Bulletin 2025/52**

(21) Application number: **24767102.7**

(22) Date of filing: **04.03.2024**

(51) International Patent Classification (IPC):
***C07H 19/20*** *(2006.01)* ***C07H 21/02*** *(2006.01)*

(52) Cooperative Patent Classification (CPC):
**C07H 19/20; C07H 21/02;** Y02P 20/55

(86) International application number:
**PCT/JP2024/007991**

(87) International publication number:
**WO 2024/185734 (12.09.2024 Gazette 2024/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.03.2023 US 202363488207 P**

(71) Applicant: **Natias Inc.**
**Kobe-shi, Hyogo 650-0047 (JP)**

(72) Inventors:
- **KATAOKA, Masanori**
  **Kobe-shi, Hyogo 650-0047 (JP)**
- **FUKUI, Chiharu**
  **Kobe-shi, Hyogo 650-0047 (JP)**
- **TSUJI, Yohei**
  **Kobe-shi, Hyogo 650-0047 (JP)**
- **FUJIMURA, Kazuma**
  **Kobe-shi, Hyogo 650-0047 (JP)**

(74) Representative: **Elzaburu S.L.P.**
**Edificio Torre de Cristal**
**Paseo de la Castellana 259 C, planta 28**
**28046 Madrid (ES)**

(54) **POLYPHOSPHORYLATED NUCLEOSIDE, PHOSPHATE-ACTIVATED NUCLEOTIDE USED FOR SYNTHESIS OF POLYPHOSPHORYLATED NUCLEOSIDE AND METHOD FOR SYNTHESIS OF SAME, AND METHOD FOR SYNTHESIS OF POLYPHOSPHORYLATED NUCLEOSIDE USING PHOSPHATE-ACTIVATED NUCLEOTIDE**

(57) The present invention provides a polyphosphorylated nucleoside having a structure of formula (I), a phosphate-activated nucleotide used for synthesizing the same and a method for synthesizing the same, and a method for synthesizing a polyphosphorylated nucleoside using the phosphate-activated nucleotide. In the formula, B is a protected or unprotected natural or artificial nucleoside base; $R^1$, $R^2$, $R^4$, and $R^5$ are each H, an alkyl group, or the like, and $R^1$, $R^2$, $R^4$, and $R^5$ may be the same or different; X and Y are each H, OH, $OCH_3$, or the like; h is an integer of 1 to 3; and p, n, m, and q are each 0 or an integer, and p, n, m, and q are in any order, with (p + n + m + q) being an integer of 0 or more and 5000 or less.

EP 4 667 479 A1

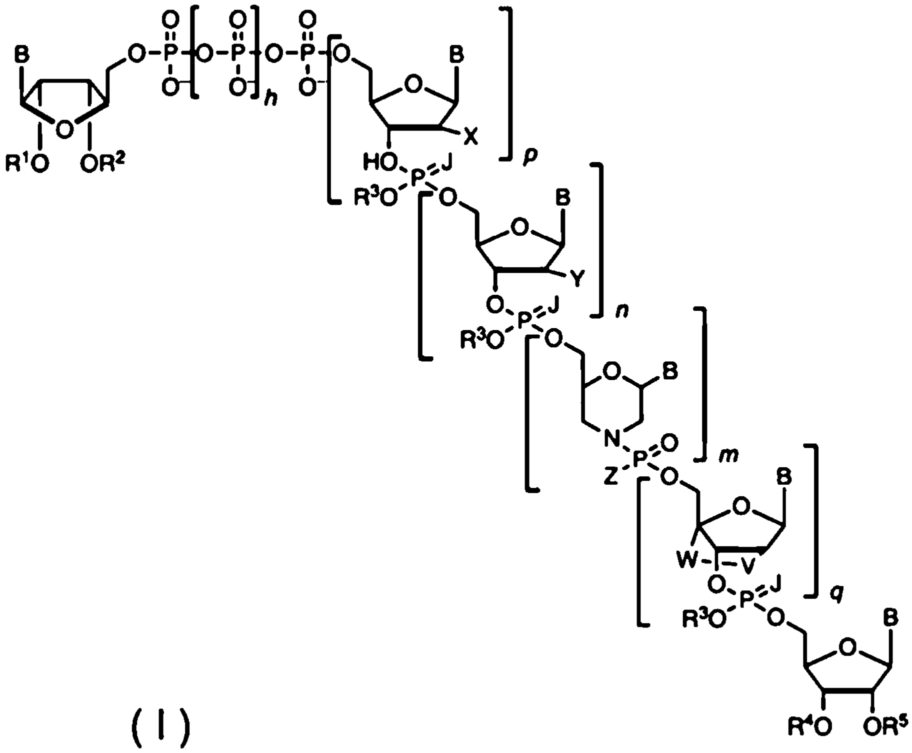

(I)

## Description

[Technical Field]

The present invention relates to a polyphosphorylated nucleoside, a phosphate-activated nucleotide used for the synthesis of polyphosphorylated nucleoside and a method for synthesizing the same, and a method for synthesizing a polyphosphorylated nucleoside using the phosphate-activated nucleotide.

[Background Art]

In recent years, the usefulness of polyphosphorylated nucleosides has been attracting attention. For example, $P^1,P^4$-di(uridine 5'-)tetraphosphate (hereinafter referred to as "$U_2P_4$") or a salt thereof, represented by the following formula (A), has expectorant activity and is expected to be a therapeutic agent for chronic obstructive pulmonary disease (PTL 1). $U_2P_4$ is also marketed as an eye drop therapeutic agent for the chronic disease of the corneal epithelium, known as dry eye, and the demands for $U_2P_4$ is high.

[Formula 1]

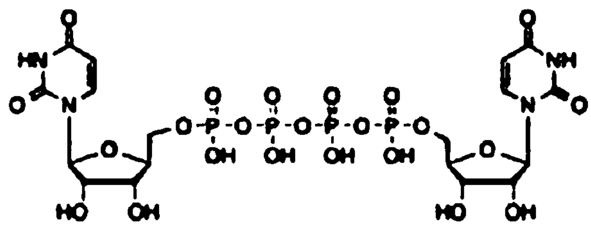

(A)

In the production of $U_2P_4$, PTL 2 adopts the following steps (1) to (3) of: (1) replacing the counter cation of the starting material uridine 5'-diphosphate with tributylammonium; (2) reacting the substituted product with carbonyl diimidazole (CDI) in DMF to imidazolidate one of the hydroxyl groups of the phosphate at the terminal side of the uridine 5'-diphosphate moiety, and (3) reacting the imidazolidated derivative with uridine 5'-diphosphate in DMF.

PTL 2 discloses another condition for the production of $U_2P_4$, where one of the hydroxyl groups of uridine 5'-diphosphate is activated with imidazole, and then the uridine 5'-diphosphate activated with imidazole is reacted with uridine 5'-diphosphate in water in the presence of a Lewis acid.

Messenger RNA (mRNA) has a triphosphorylated nucleoside structure, known as a cap structure, at the 5' end. PTL 3 discloses an example of producing a cap structure by a method of activating one of the hydroxyl groups of nucleoside 5'-phosphate with imidazole as described above.

PTL 4 discloses a method of performing a coupling reaction between a nucleoside 5'-monophosphate and a nucleoside 5'-diphosphate activated at one of the hydroxyl groups with imidazole in the presence of a Lewis acid catalyst, using DMF as the reaction solvent, during the synthesis of triphosphate nucleosides.

[Citation List]

[Patent Literature]

[PTL 1] PCT International Publication No. WO 2008/012949
[PTL 2] PCT International Publication No. WO 2014/103704
[PTL 3] PCT International Publication No. WO 2017/053297
[PTL 4] PCT International Publication No. WO 2022/212442

[Summary of Invention]

[Technical Problem]

All of the aforementioned production methods use nucleoside 5'-diphosphate as the starting material, but nucleoside 5'-diphosphate is expensive. Furthermore, all of the production methods include the production of nucleoside

triphosphate or nucleoside tetraphosphate using a condensing agent or an activating agent, thereby requiring multiple steps.

Furthermore, since the reaction efficiency at each stage is not necessarily high, the above production methods require considerable time for purifying the crude product and concentrating the solution obtained from purification. As a result, the above production methods have low product yield, and poor time efficiency and cost performance of the production.

The present invention has been made in view of such circumstances, and an object of the present invention is to provide a polyphosphorylated nucleoside that can be efficiently produced using less expensive raw materials through a short process with simple procedures, a phosphate-activated nucleotide used for the synthesis of polyphosphorylated nucleoside and a method for synthesizing the same, and a method for synthesizing a polyphosphorylated nucleoside using the phosphate-activated nucleotide.

[Solution to Problem]

To solve the above problem, the polyphosphorylated nucleoside, the phosphate-activated nucleotide used for the synthesis of polyphosphorylated nucleotide and the method for synthesizing the same, and the method for synthesizing a polyphosphorylated nucleoside using the phosphate-activated nucleotide according to the present invention employ the following means.

The first aspect of the present invention provides a polyphosphorylated nucleoside having a structure of formula (I):

[Formula 2]

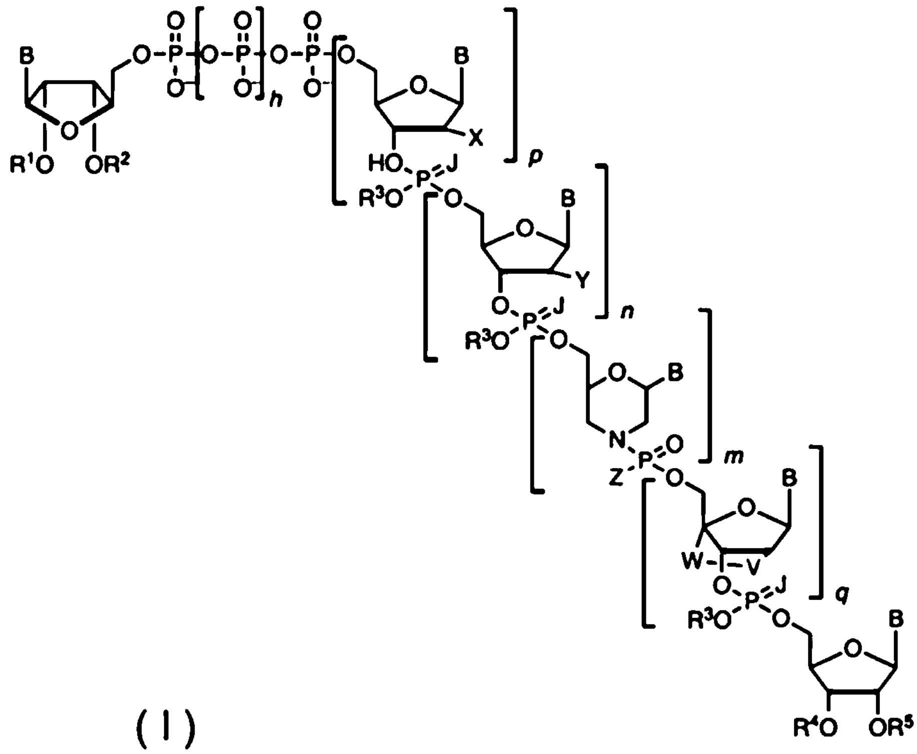

(I)

wherein

B is independently a natural or artificial nucleoside base that is protected by a protecting group or unprotected;

$R^1$ and $R^2$ are each independently H, $CH_2CH=CH_2$, $CH_2C_6H_5$, $CH_2C_6H_4$-p-$OCH_3$, 2-$CH_2C_{10}H_7$, 1-pyrenylmethyl, acetyl, benzoyl, dichloroacetyl, 1-pentenoyl, levulinoyl, phenoxyacetyl, $CONHC_6H_5$, $CONHC_{10}H_7$, Boc, Fmoc, allyloxycarbonyl, benzyloxycarbonyl, TOM, Pivom, CEM, $CH_2OCH_2C_6H_4$-p-$OCH_3$ (PMBOM), or $CH_2O$-2-$CH_2C_{10}H_7$ (NAPOM), and $R^1$ and $R^2$ may be the same or different;

$R^3$ is independently methyl, ethyl, a vinyl derivative, an allyl derivative, isopropyl, a phenyl derivative, or a polyfluoroalkyl derivative;

$R^4$ and $R^5$ are each independently H, $CH_2CH=CH_2$, $CH_2C_6H_5$, $CH_2C_6H_4$-p-$OCH_3$, 2-$CH_2C_{10}H_7$, 1-pyrenylmethyl, acetyl, benzoyl, dichloroacetyl, 1-pentenoyl, levulinoyl, phenoxyacetyl, $CONHC_6H_5$, $CONHC_{10}H_7$, Boc, Fmoc, allyloxycarbonyl, benzyloxycarbonyl, TOM, Pivom, CEM, $CH_2OCH_2C_6H_4$-p-$OCH_3$ (PMBOM), or $CH_2O$-2-$CH_2C_{10}H_7$ (NAPOM), and $R^4$ and $R^5$ may be the same or different;
X and Y are each independently H, OH, $OCH_3$, $OCH_2CH_2OCH_3$, or F;
J is independently O, S, Se, or BH;
Z is OH, $NH_2$, or $N(CH_3)_2$;
W is $CH_2$, $CH_2CH_2$, or $CH(CH_3)$;
V is O, $NCH_3$, NCOR, or N-N=NH;
h is an integer of 1 or more and 3 or less; and
p, n, m, and q are each 0 or an integer, and p, n, m, and q are in any order, with (p + n + m + q) being an integer of 0 or more and 5000 or less.

In the first aspect, $R^1$ and $R^2$ nay be each a dichloroacetyl group, and $R^3$ may be H or a metal salt.

In the first aspect, $R^1$ and $R^2$ may be each a phenoxyacetyl group, and $R^3$ may be H or a metal salt.

The second aspect of the present invention provides 5'-diphosphate-activated nucleotide used to produce the polyphosphorylated nucleoside according to the first aspect, wherein the 5'-diphosphate-activated nucleotide has a structure of the following formula (II):

[Formula 3]

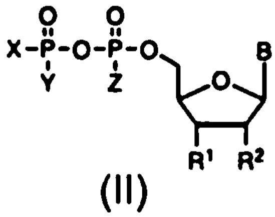

(II)

wherein

B is independently a natural or artificial nucleoside base that is protected by a protecting group or unprotected;
X, Y, and Z are each independently OH, $OR^6$, SH, SR, $BH_3$, halogen, azolide, $NR_2$, or $NH_2$;
$R^1$ and $R^2$ are each independently H, OH, halogen, an alkyl group, a hydroxyl group protected by an acyl-based protecting group, a carbamoyl-based protecting group or a silyl-based protecting group, or a 3 to 11-mer oligonucleotide having a natural or artificial nucleoside base that is unprotected or protected by a protecting group, and $R^1$ and $R^2$ may be the same or different; and
$R^6$ is OBT, OAt, a substituted or unsubstituted vinyl group, or a substituted or unsubstituted allyl group, wherein the substituted vinyl group may contain a cyclic structure.

The third aspect of the present invention provides a method for synthesizing the 5'-diphosphate-activated nucleotide according to the second aspect, the method comprising steps of:

[Formula 4]

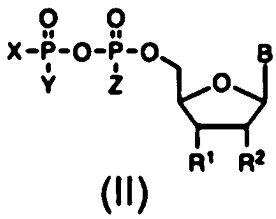

(II)

(a) reacting pyrophosphoryl chloride with a co-catalyst compound in a solvent, in the presence or absence of a catalyst compound to prepare a phosphate-activated compound; and
(b) reacting the phosphate-activated compound obtained in step (a) with a nucleoside in which a base moiety is protected by a protecting group or unprotected to prepare a 5'-diphosphate-activated nucleotide, wherein in formula

(II), X = Y = Cl, Z = Cl or $O^-$.

The fourth aspect of the present invention provides a method for synthesizing the 5'-diphosphate-activated nucleotide according to the second aspect, the method comprising steps of:

[Formula 5]

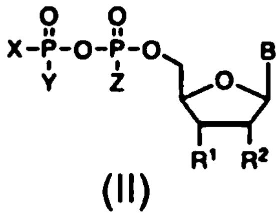

(II)

(a) reacting a polychlorinating agent with a co-catalyst compound in the presence or absence of a catalyst compound to prepare a phosphate-activated compound; and
(b) reacting the phosphate-activated compound obtained in step (a) with a nucleoside 5'-monophosphate in which a base moiety is protected by a protecting group or unprotected, in a water-hydrophilic organic solvent to prepare a 5'-diphosphate-activated nucleotide, wherein in the formula, X = Y = azolide or a heterocyclic compound derivative, and Z = azolide, a heterocyclic compound derivative, or $O^-$.

The fifth aspect of the present invention provides a method for synthesizing the 5'-diphosphate-activated nucleotide according to the second aspect, the method comprising steps of:

[Formula 6]

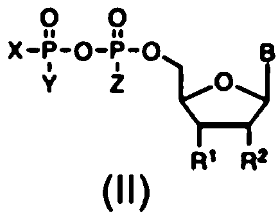

(II)

(a) reacting a polychlorinating agent with a nucleoside 5'-diphosphate in which a base moiety is protected by a protecting group or unprotected, in a water-hydrophilic organic solvent in the presence or absence of a catalyst compound to prepare a nucleoside 5'-diphosphate chloride, wherein in the formula, X = Y = Cl; and
(b) adding a co-catalyst compound to a reaction liquid after step (a) to obtain a 5'-diphosphate-activated nucleotide, wherein in the formula, X = Y = azolide or a heterocyclic compound derivative, and Z = azolide, a heterocyclic compound derivative, or $O^-$.

In the third to fifth aspects, the co-catalyst compound may be an imidazole or a benzimidazole.

In the third to fifth aspects, the catalyst compound may be an organic catalyst that is HOBT, HOAu, Oxyma (ethyl isonitrosocyanoacetate), or DMT-MM, a Lewis acid such as iron(III) chloride, aluminum chloride, zinc chloride, copper(II) chloride, magnesium chloride, or cesium chloride, cerium triflate, or scandium triflate.

In the fourth or fifth aspect, the polychlorinating agent may be phosphoryl chloride, dichlorophosphoric anhydride, phosphorus pentachloride, thionyl chloride, or 2-chloro-1,3-dimethylimidazolinium chloride.

The sixth aspect of the present invention provides a method for synthesizing a polyphosphorylated nucleoside according to the first aspect, the method comprising a step of:
condensing the 5'-diphosphate-activated nucleotide according to the third to fifth aspects with a nucleoside 5'-phosphate in the presence of water to obtain a polyphosphorylated nucleoside.

In the sixth aspect, the nucleoside 5'-phosphate may be a 5'-monophosphorylated nucleic acid selected from guanosine 5'-phosphate, N7-methylguanosine 5'-phosphate, RNA, DNA, a DNA/RNA chimera, or a modified nucleoside-containing RNA.

[Advantageous Effects of Invention]

According to the method for synthesizing a polyphosphorylated nucleoside of the present invention, it is possible to produce the polyphosphorylated nucleoside of interest using less expensive raw materials with simple procedures. In addition, it is possible to obtain the target product efficiently through a short process.

According to the 5'-diphosphate-activated nucleotide used in the synthesis of the polyphosphorylated nucleoside of the present invention, the reaction can proceed using only water or an organic solvent containing water as a solvent. This enables the synthesis method to reduce environmental impact.

[Brief Description of Drawings]

[Fig. 1]
Fig. 1 shows the HPLC charts of $G_2P_3$ synthesized by the synthesis method according to the present embodiment before purification (a) and after purification (b) by anion exchange column chromatography.
[Fig. 2]
Fig. 2 shows the HPLC chart of the crude products after the reaction for synthesizing $U_2P_4$ using uridine 5'-diphosphate as the starting material in Example 1 of the present embodiment.
[Fig. 3]
Fig. 3 shows the MS spectra for the synthesis of $U_2P_4$ using uridine 5'-diphosphate as the starting material in Example 2 of the present embodiment. MS spectra of reaction solution, $U_2P_4$, UDP, and uridine monophosphate are shown from top to bottom.
[Fig. 4]
Fig. 4 shows the HPLC chart of the crude products obtained after the synthesis reaction in Example 2 of the present embodiment.
[Fig. 5]
Fig. 5 shows the preparative LC chromatogram of the crude products obtained after the synthesis reaction in Example 2 of the present embodiment.
[Fig. 6]
Fig. 6 shows the HPLC chart of $U_2P_4$ obtained by freeze-drying the Peak 4 fraction solution in Fig. 5.
[Fig. 7]
Fig. 7 shows the HPLC chart of the crude products obtained after the reaction in Example 3 of the present embodiment.
[Fig. 8]
Fig. 8 shows the HPLC chart of the crude products obtained after the synthesis reaction in Example 5 of the present embodiment.

[Description of Embodiments]

Hereinafter, one embodiment of the polyphosphorylated nucleoside, the phosphate-activated nucleotide used for the synthesis of polyphosphorylated nucleoside and the method for synthesizing the same, and the method for synthesizing a polyphosphorylated nucleoside using the phosphate-activated nucleoside according to the present invention will be described.

The polyphosphorylated nucleoside in the present embodiment has the structure of the following formula (I):

[Formula 7]

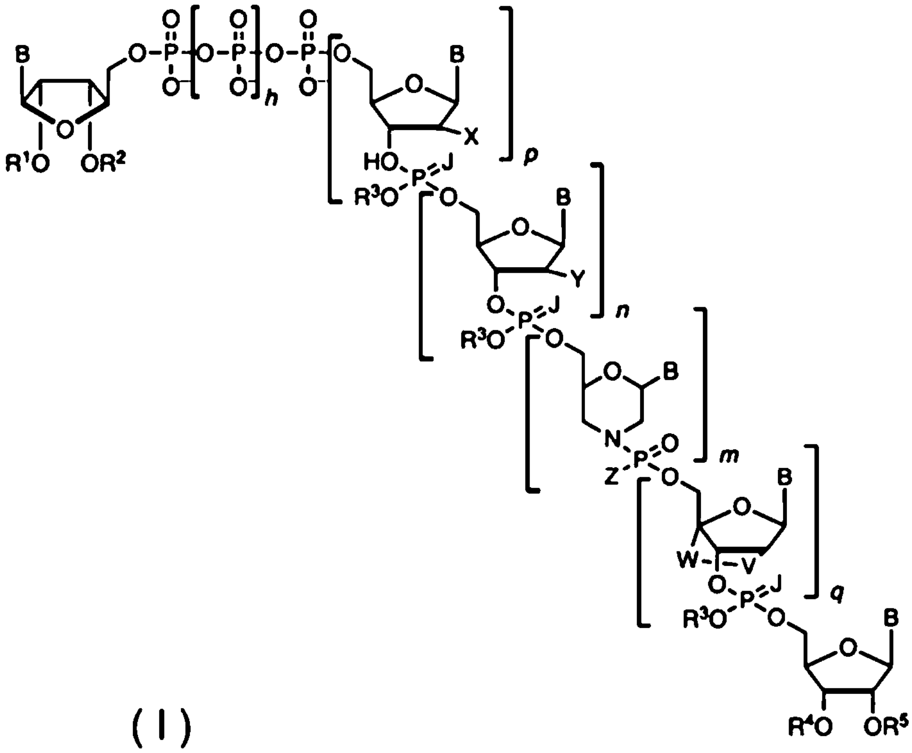

( I )

wherein

B is independently a natural or artificial nucleoside base that is protected by a protecting group or unprotected;
$R^1$ and $R^2$ are each independently H, $CH_2CH=CH_2$, $CH_2C_6H_5$, $CH_2C_6H_4$-p-$OCH_3$, 2-$CH_2C_{10}H_7$, 1-pyrenylmethyl, acetyl, benzoyl, dichloroacetyl, 1-pentenoyl, levulinoyl, phenoxyacetyl, a fatty acid ester with 5 to 22 carbon atoms, an alkyl group with 3 to 11 carbon atoms containing an alkyne moiety, a biotin ester, $CONHC_6H_5$, $CONHC_{10}H_7$, Boc, Fmoc, allyloxycarbonyl, benzyloxycarbonyl, TOM, Pivom, CEM, $CH_2OCH_2C_6H_4$-p-$OCH_3$ (PMBOM), or $CH_2O$-2-$CH_2C_{10}H_7$ (NAPOM), and $R^1$ and $R^2$ may be the same or different;
$R^3$ is independently methyl, ethyl, a vinyl derivative, an allyl derivative, isopropyl, a phenyl derivative, or a polyfluoroalkyl derivative;
$R^4$ and $R^5$ are each independently H, $CH_2CH=CH_2$, $CH_2C_6H_5$, $CH_2C_6H_4$-p-$OCH_3$, 2-$CH_2C_{10}H_7$, 1-pyrenylmethyl, acetyl, benzoyl, dichloroacetyl, 1-pentenoyl, levulinoyl, phenoxyacetyl, $CONHC_6H_5$, $CONHC_{10}H_7$, Boc, Fmoc, allyloxycarbonyl, benzyloxycarbonyl, TOM, Pivom, CEM, $CH_2OCH_2C_6H_4$-p-$OCH_3$ (PMBOM), or $CH_2O$-2-$CH_2C_{10}H_7$ (NAPOM), and $R^4$ and $R^5$ may be the same or different;
$R^3$ is independently methyl, ethyl, a vinyl derivative, an allyl derivative, isopropyl, a phenyl derivative, or a polyfluoroalkyl derivative;
X and Y are each independently H, OH, $OCH_3$, $OCH_2CH_2OCH_3$, or F;
J is independently O, S, Se, or BH;
Z is OH, $NH_2$, or $N(CH_3)_2$;
W is $CH_2$, $CH_2CH_2$, or $CH(CH_3)$;
V is O, $NCH_3$, NCOR, or N-N=NH;
h is an integer of 1 or more and 3 or less; and
p, n, m, and q are each 0 or an integer, and p, n, m, and q are in any order, with (p + n + m + q) being an integer of 0 or more and 5,000 or less.

In the present description, the nucleoside located at the 5'-most end in formula (I) is referred to as "left wing", and the nucleoside or nucleotide having a polyphosphate bound to the 3'-hydroxyl group of the left wing at the 5'-end is referred to as the "right wing". In the left wing, both the 2'- and 3'-hydroxyl groups of the nucleoside may be protected by an acyl group.

Examples of the acyl group include, but are not limited to, an acetyl group, a benzoyl group, a dichloroacetyl group, a 1-pentenoyl group, a levulinoyl group, a phenoxyacetyl group, and a fatty acid esters with 5 to 22 carbon atoms.

In the right wing, the sugars possessed by the nucleosides may consist entirely of natural pentoses (ribose or 2'-deoxyribose), or may include morpholino nucleoside.

The right wing may have an oligonucleotide structure in which multiple nucleotides are bound. In formula (I), p, n, m, and q are in any order. p, n, m, and q are each 0 or an integer, and (p + n + m + q) is an integer of 0 or more and 5,000 or less. (p + n + m + q) is preferably 0 or more and 200 or less, more preferably 0 or more and 50 or less, and further preferably 0 or more and 25 or less.

B in formula (I) is a natural or artificial nucleoside base. The base moiety may also be protected by a protecting group. The base moiety may be unprotected. The protecting group may include a labeled functional group. Examples of labeling include, but are not limited to, labeling with a fluorescent functional group, labeling with a radioactive isotope, labeling with a stable isotope, and labeling with biotin.

Examples of the protecting group for the base moiety include, but are not limited to, an acyl-based protecting group, an alkyloxy protecting group, a carbamoyl protecting group, and an amidine protecting group.

Examples of the protecting group for the phosphate moiety include methyl, ethyl, a vinyl derivative, an allyl derivative, isopropyl, a phenyl derivative, and a polyfluoroalkyl derivative.

$R^1$ and $R^2$ positioned at the 2' or 3' position of the nucleoside of the left wing may be each H, $CH_2CH=CH_2$, $CH_2C_6H_5$, $CH_2C_6H_4$-p-$OCH_3$, 2-$CH_2C_{10}H_7$, 1-pyrenylmethyl, acetyl, benzoyl, dichloroacetyl, 1-pentenoyl, levulinoyl, phenoxyacetyl, a fatty acid ester with 5 to 22 carbon atoms, an alkyl group with 3 to 11 carbon atoms containing an alkyne moiety, a biotin ester, $CONHC_6H_5$, $CONHC_{10}H_7$, Boc, Fmoc, allyloxycarbonyl, benzyloxycarbonyl, TOM, Pivom, CEM, $CH_2OCH_2C_6H_4$-p-$OCH_3$ (PMBOM), or $CH_2O$-2-$CH_2C_{10}H_7$ (NAPOM). Examples of alkyl group include $OCH_2CH=CH_2$, $CH_2C_6H_5$, $CH_2C_6H_4$-p-$OCH_3$, 2-$CH_2C_{10}H_7$, and 1-pyrenylmethyl. Examples of acyl group include an acetyl group, a benzoyl group, a dichloroacetyl group, a 1-pentenoyl group, a levulinoyl group, and a phenoxyacetyl group. Examples of carbamoyl group include $CONHC_6H_5$ and $CONHC_{10}H_7$. Other examples include carbonates such as Boc and Fmoc, allyloxycarbonyl, and benzyloxycarbonyl. Examples of acetal-type protecting group include TOM, Pivom, CEM, $CH_2OCH_2C_6H_4$-p-$OCH_3$ (PMBOM), and $CH_2O$-2-$CH_2C_{10}H_7$ (NAPOM). Examples of silyl-based protecting group include a trimethylsilyl group, a triethylsilyl group, a t-butyldimethylsilyl group, a t-butyldiphenylsilyl group, a tetraisopropyldisiloxy group, and di-t-butylsilyl. $R^1$ and $R^2$ may be the same or different within a single molecule.

$R^4$ and $R^5$ positioned at the 2' or 3' position of the nucleoside at the 3' end of the right wing may be each H, $CH_2CH=CH_2$, $CH_2C_6H_5$, $CH_2C_6H_4$-p-$OCH_3$, 2-$CH_2C_{10}H_7$, 1-pyrenylmethyl, acetyl, benzoyl, dichloroacetyl, 1-pentenoyl, levulinoyl, phenoxyacetyl, $CONHC_6H_5$, $CONHC_{10}H_7$, Boc, Fmoc, allyloxycarbonyl, benzyloxycarbonyl, TOM, Pivom, CEM, $CH_2OCH_2C_6H_4$-p-$OCH_3$ (PMBOM), or $CH_2O$-2-$CH_2C_{10}H_7$ (NAPOM). Examples of the alkyl group include $CH_2CH=CH_2$, $CH_2C_6H_5$, $CH_2C_6H_4$-p-$OCH_3$, 2-$CH_2C_{10}H_7$, and 1-pyrenylmethyl. Examples of the acyl group include an acetyl group, a benzoyl group, a dichloroacetyl group, a 1-pentenoyl group, a levulinoyl group, and a phenoxyacetyl group. Examples of carbamoyl group include $CONHC_6H_5$ and $CONHC_{10}H_7$. Other examples include a carbonate such as Boc or Fmoc, allyloxycarbonyl, and benzyloxycarbonyl. Examples of acetal-type protecting group include TOM, Pivom, CEM, $CH_2OCH_2C_6H_4$-p-$OCH_3$ (PMBOM), and $CH_2O$-2-$CH_2C_{10}H_7$ (NAPOM). Examples of silyl-based protecting group include a trimethylsilyl group, a triethylsilyl group, a t-butyldimethylsilyl group, a t-butyldiphenylsilyl group, a tetraisopropyldisiloxy group, and di-t-butylsilyl. $R^4$ and $R^5$ may be the same or different within a single molecule.

Examples of known polyphosphorylated nucleoside include Diquafosol (structural formula $U_2P_4$), which is used as an ingredient in eye drops, and the cap structure of mRNA that has guanosine methylated at the 7-position ($m^7G$) on the left side through a triphosphate moiety.

The polyphosphorylated nucleoside according to the present embodiment is different from these known substances in that a protecting group is introduced at the 2'-hydroxyl group and/or 3'-hydroxyl group of the ribose ring of the left wing. The polyphosphorylated nucleoside according to the present embodiment can achieve high double-strand formation capability and lipo-solubility due to having such a configuration. In addition, the reaction sites are protected by protecting the hydroxyl group, resulting in high reactivity and selectivity.

In the synthesis of the polyphosphorylated nucleoside in the present embodiment, a nucleoside with an activated 5'-diphosphate moiety is used as a synthetic intermediate.

[Formula 8]

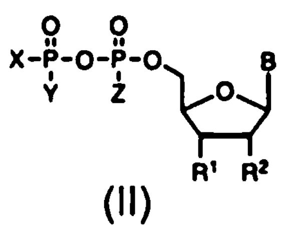

(II)

wherein

B is independently a natural or artificial nucleoside base that is protected by a protecting group or unprotected;
X, Y, and Z are each independently OH, $OR^6$, SH, SR, $BH_3$, halogen, azolide, $NR_2$, or $NH_2$;
$R^1$ and $R^2$ are each independently H, OH, halogen, an alkyl group, a hydroxyl group protected by an acyl-based protecting group, a carbamoyl-based protecting group or a silyl-based protecting group, or a 3 to 11-mer oligonucleotide having a natural or artificial nucleoside base that is unprotected or protected by a protecting group, and $R^1$ and $R^2$ may be the same or different; and
$R^6$ is OBT, OAt, a substituted or unsubstituted vinyl group, or a substituted or unsubstituted allyl group, wherein the substituted vinyl group may include a cyclic structure.

The bases in the present embodiment include all bases of naturally occurring nucleosides (hereinafter referred to as "natural bases"). The most common bases in naturally occurring nucleosides are purines and pyrimidines. Examples of naturally occurring purine rings include adenine, guanine, and N6-methyladenine. Examples of naturally occurring pyrimidines include cytosine, thymine, 5-methylcytosine, and pseudouracil. Examples of natural nucleosides include ribose of adenosine, guanosine, cytidine, thymidine, uridine, inosine, 7-methylguanosine, or pseudouridine, and 2'-O-methyl or 2'-deoxyribo derivatives.

The base in the present embodiment may be an unnatural base or an artificial base. The unnatural base or artificial base refers to an artificially synthesized base analog that has properties similar to natural bases and can pair with a complementary base analogue (also referred to as a "complementary artificial base"), like natural bases.

The synthesis method of 5'-diphosphate-activated nucleotide includes three types of synthetic routes: (1) a synthetic route using a nucleoside as the starting material, (2) a synthetic route using a nucleoside 5'-monophosphate as the starting material, and (3) a synthetic route using a nucleoside 5'-diphosphate as the starting material. Hereinafter, the outline of each synthetic route will be described.

In synthetic routes (1) and (2), first, a phosphorylating agent is activated in the initial stage, and reacted with a nucleoside or a nucleoside monophosphate in the next stage.

(1) Synthetic route using nucleoside as starting material

In this route, a phosphorylating agent, pyrophosphoryl chloride, is reacted with a co-catalyst compound in a solvent in the presence or absence of a catalyst compound to prepare a phosphate-activated compound. Subsequently, the obtained phosphate-activated compound is reacted with a nucleoside in which a base moiety is protected by a protecting group or unprotected, in a hydrophilic organic solvent or a water-hydrophilic organic solvent to obtain a 5'-diphosphate-activated nucleotide of interest, wherein in formula (II), X = Y = Cl, Z = Cl or $O^-$.

Examples of the co-catalyst compound used in synthetic route (1) include imidazole or benzimidazole, with benzimidazole being preferred.

Examples of the catalyst compound used in synthetic route (1) include an organic catalyst that is HOBT, HOAT, HOCU, or DMT-MM, a Lewis acid such as iron(III) chloride, aluminum chloride, zinc chloride, copper(II) chloride, magnesium chloride, or cesium chloride, and an inorganic or organic salt such as cerium triflate or scandium triflate. The catalyst compound is not essential, and it is possible to prepare the phosphate-activated compound even without adding the catalyst compound to the reaction solution. When adding the catalyst compound, it is preferable that the catalyst compound in synthetic route (1) is a Lewis acid, and more preferably iron(III) chloride.

In synthetic route (1), it is preferable to use each of 0.3 to 5.0 equivalents of the catalyst compound, 1.0 to 5.0 equivalents of the co-catalyst compound per hydroxyl group of the phosphate moiety, and 1.0 to 5.0 equivalents of the polychlorinating agent per hydroxyl group of the phosphate moiety.

The synthesis in synthetic route (1) can be performed in a hydrophilic organic solvent or a hydrophilic organic solvent containing water. As the hydrophilic organic solvent, trimethyl phosphate is preferred. The reaction temperature is preferably -30°C to 30°C, and more preferably 0°C, in the stage of preparing the phosphate-activated compound. In the stage after adding the nucleoside, the reaction temperature is preferably 10°C to 70°C, and more preferably 50°C

Examples of the hydrophilic organic solvent used in the synthesis according to synthetic route (1) include trimethyl phosphate, trimethyl phosphite, acetonitrile, THF, dimethyl carbonate, and DMF, with acetonitrile being preferred.

(2) Synthetic route using nucleoside 5'-monophosphate as starting material

In this route, a polychlorinating agent is reacted with a co-catalyst compound in a solvent in the presence or absence of a catalyst compound to prepare a phosphate-activated compound. The obtained phosphate-activated compound is then reacted with a nucleoside 5'-monophosphate in which a base moiety is protected by a protecting group or unprotected, in a hydrophilic organic solvent or a water-hydrophilic organic solvent to prepare a 5'-diphosphate-activated nucleotide of interest, wherein in formula (II), X = Y = azolide or a heterocyclic compound derivative, and Z =

azolide, a heterocyclic compound derivative, or $O^-$. Examples of the azolide include imidazolide, benzimidazolide, 1,2,4-triazolide, tetrazolide, pyrazolide, and a derivative thereof. Examples of the heterocyclic compound derivative include pyridinium, 4-N,N'-dimethylaminopyridinium, and a derivative thereof.

Examples of the polychlorinating agent used in synthetic route (2) include phosphoryl chloride, dichlorophosphoric anhydride, thionyl chloride, or 2-chloro-1,3-dimethylimidazolinium chloride. Dichlorophosphoric anhydride forms a diphosphate-type activated compound when mixed with the catalyst compound Lewis acid, but under certain reaction conditions, the main product becomes a nucleoside monophosphate with only one phosphate added.

Examples of the co-catalyst compound used in synthetic route (2) include imidazole or benzimidazole, with imidazole being preferred.

Examples of the catalyst compound used in synthetic route (2) include an organic catalyst that is HOBT, HOAu, HOCU, or DMT-MM, a Lewis acid such as iron(III) chloride, aluminum chloride, zinc chloride, copper(II) chloride, magnesium chloride, or cesium chloride, and an inorganic or organic salt such as cerium triflate or scandium triflate. The catalyst compound is not essential, and it is possible to prepare the phosphate-activated compound even without adding the catalyst compound to the reaction solution. When adding the catalyst compound, it is preferable that the catalyst compound in synthetic route (2) is a Lewis acid, and more preferably iron(III) chloride.

In synthetic route (2), it is preferable to use each of 0.3 to 5.0 equivalents of the catalyst compound, 1.0 to 5.0 equivalents of the co-catalyst compound per hydroxyl group of the phosphate moiety, and 1.0 to 5.0 equivalents of the polychlorinating agent per hydroxyl group of the phosphate moiety.

The synthesis in synthetic route (2) can be performed in a hydrophilic organic solvent, a hydrophilic organic solvent containing water, or water. The reaction temperature is preferably 10°C to 30°C in the stage of preparing the phosphate-activated compound, allowing the phosphate-activated compound of interest to be obtained even at room temperature. In the stage after adding nucleoside 5'-monophosphate, the reaction temperature is preferably 0°C to 70°C, with 25°C being more preferable.

Examples of the hydrophilic organic solvent used in the synthesis in synthetic route (2) include acetonitrile, THF, dimethyl carbonate, and DMF, with acetonitrile being preferred.

(3) Synthetic route using nucleoside 5'-diphosphate as starting material

In this route, a polychlorinating agent is reacted with a nucleoside 5'-diphosphate in which a base moiety is protected by a protecting group or unprotected, in a solvent in the presence or absence of a catalyst compound to prepare a nucleoside 5'-diphosphate chloride, wherein in formula (II), X = Y = Cl. The obtained nucleoside 5'-diphosphate chloride is then reacted with a co-catalyst compound in a hydrophilic organic solvent, a water-hydrophilic organic solvent, or water to prepare a 5'-diphosphate-activated nucleotide of interest, wherein in formula (II), X = Y = azolide or a heterocyclic compound derivative, and Z = azolide, a heterocyclic compound derivative, or $O^-$. The pH of the aqueous solution is preferably 1 to 5, and more preferably 2. Examples of the azolide include imidazolide, benzimidazolide, 1,2,4-triazolide, tetrazolide, pyrazolide, and a derivative thereof. Examples of the heterocyclic compound derivative include pyridinium, 4-N,N'-dimethylaminopyridinium, and a derivative thereof.

Examples of the polychlorinating agent used in synthetic route (3) include phosphoryl chloride, dichlorophosphoric anhydride, phosphorus pentachloride, thionyl chloride, or 2-chloro-1,3-dimethylimidazolinium chloride, and Vilsmeier reagent, with 2-chloro-1,3-dimethylimidazolinium chloride being preferred. Dichlorophosphoric anhydride forms a diphosphate-type activated compound when mixed with the catalyst compound Lewis acid, but under certain reaction conditions, the main product becomes a nucleoside monophosphate with only one phosphate added. In contrast, 2-chloro-1,3-dimethylimidazolinium chloride enables chlorination without the addition of phosphate, thus can be used when chlorinating nucleoside diphosphate. To promote chlorination, a condensing agent can be used in combination. Examples of the condensing agent include an uronium-type condensing agent such as DMT-MM and COMU, a carbodiimide-type condensing agent such as DCC, DIC, or EDC, and a phosphonium-type condensing agent such as Py-BOP.

Examples of the co-catalyst compound used in synthetic route (3) include imidazole or benzimidazole, with benzimidazole being preferred. Due to the high acidity and steric hindrance of benzimidazole, the reactivity and selectivity in synthetic route (3) are enhanced.

Examples of the catalyst compound used in synthetic route (3) include an organic catalyst that is HOBT, HOAT, or Oxyma, a Lewis acid such as iron(III) chloride, aluminum chloride, zinc chloride, copper(II) chloride, magnesium chloride, or cesium chloride, and an inorganic or organic salt such as cerium triflate or scandium triflate. The catalyst compound is not essential, and it is possible to prepare the phosphate-activated compound even without adding the catalyst compound to the reaction solution. When adding the catalyst compound, it is preferable that the catalyst compound in synthetic route (3) is a Lewis acid, and more preferably iron(III) chloride.

In synthetic route (3), it is preferable to use each of 0.3 to 5.0 equivalents of the catalyst compound, 1.0 to 5.0 equivalents of the co-catalyst compound per hydroxyl group of the phosphate moiety, and 1.0 to 5.0 equivalents of the

polychlorinating agent per hydroxyl group of the phosphate moiety.

The synthesis in synthetic route (3) can be performed in a hydrophilic organic solvent, a hydrophilic organic solvent containing water, or water. The reaction temperature is preferably -30°C to 30°C, and more preferably 25°C, in the stage of preparing the phosphate-activated compound. In the stage after adding the co-catalyst compound, the reaction temperature is preferably 10°C to 70°C, and more preferably 25°C. The pH of the aqueous solution is preferably 1 to 5, and more preferably 3.

Examples of the hydrophilic organic solvent used in the synthesis of synthetic route (3) include acetonitrile, trimethyl phosphate, trimethyl phosphite, THF, dimethyl carbonate, DMF, and DMSO, with acetonitrile being preferred.

The synthesized 5'-diphosphate-activated nucleotide according to the present embodiment can be confirmed in the reaction system by LC-MS or $^{31}$P-NMR.

The method for synthesizing polyphosphorylated nucleoside using 5'-phosphate-activated nucleotide according to the present embodiment will be explained. The method includes the step of condensing a 5'-diphosphate-activated nucleotide obtained from any of synthetic routes (1) to (3) described above with a nucleoside 5'-phosphate in the presence of water to obtain a polyphosphorylated nucleoside.

In the method for synthesizing a polyphosphorylated nucleoside using a 5'-phosphate-activated nucleotide according to the present embodiment, the nucleoside 5'-phosphate used may be a 5'-monophosphorylated nucleic acid selected from guanosine 5'-phosphate, N7-methylguanosine 5'-phosphate, RNA, DNA, a DNA/RNA chimera, or a modified nucleoside-containing RNA.

In the synthesis method of the present embodiment, it is possible to synthesize a phosphate-activated nucleoside using a commercially available and inexpensive chlorinating agent or polychlorinating agent. This allows for the procurement of raw materials at a lower cost compared to conventional synthesis methods by minimizing the synthetic route using nucleoside or monophosphate as the starting material.

In the synthesis method of the present embodiment, polyphosphorylated nucleoside is synthesized via 5'-diphosphate-activated nucleotide. The 5'-diphosphate-activated nucleotide according to the present embodiment can efficiently construct polyphosphate when water is used as the solvent for polyphosphate synthesis. In this case, positional selectivity, functional group selectivity, and reactivity are high, while side reactions are minimal.

Furthermore, according to the synthesis method of the present embodiment, because there are fewer steps and the reaction system is simpler compared to conventional methods, the purification process can be more convenient.

According to the present embodiment, it is possible to synthesize a 5'-diphosphate-activated nucleotide and synthesize a polyphosphorylated nucleoside in water or a hydrophilic organic solvent containing water. This allows for the recovery of the nucleoside monophosphate and nucleoside diphosphate used as raw materials, enabling their reuse in synthesis.

According to the synthesis method of polyphosphorylated nucleoside of the present embodiment, it is possible to synthesize a polyphosphorylated nucleoside with a symmetrical structure by converting a single raw material into an activated diphosphorylated nucleoside.

Furthermore, according to the synthesis method of polyphosphorylated nucleoside of the present embodiment, a single raw material is converted into an activated diphosphorylated nucleotide, which is then reacted with a monophosphorylated or diphosphorylated nucleotide. Asymmetric polyphosphorylated nucleoside can be synthesized by adding a solution of non-activated phosphorylated nucleoside to activated diphosphorylated nucleotide (0.1 to 5.0 equivalents, preferably 1.0 equivalent of non-activated phosphorylated nucleoside with respect to activated diphosphorylated nucleotide) before forming a symmetrical dimer.

According to the synthesis method of polyphosphorylated nucleoside of the present embodiment, the target product can be obtained by affinity-purification with biotinylation of either $R^1$ or $R^2$.

As an example of the polyphosphorylated nucleoside, the synthesis method of $G_2P_3$ using guanosine as the starting material will be explained.

[Formula 9]

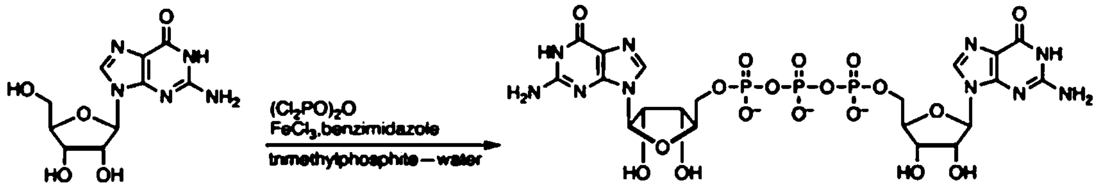


Iron(III) chloride (162 mg, 1.0 mmol) is suspended in trimethyl phosphate (2.0 mL). To the solution, pyrophosphoryl chloride (138 μL, 1.0 mmol) is slowly added dropwise at 0°C, and the mixture is stirred for 20 minutes. Guanosine (113 mg, 0.4 mmol) is added gradually in small portions, and the mixture is stirred for 10 minutes. A solution of

benzimidazole (307 mg, 2.6 mmol) in acetonitrile (0.8 mL) is added dropwise to the reaction solution at room temperature over 5 minutes. After the mixture is stirred at 50°C for 10 minutes, 200 μL of deionized water is added dropwise 10 times (a total of 2 mL), and the mixture is stirred for 10 minutes. A viscous crude product is obtained from the lower layer.

The crude product is diluted with deionized water (50 mL) and purified using an ion exchange column (YMC Co., BioPro IEX SmartSep Q). For elution, solution A: 0.05 M $NH_4HCO_3$ and solution B: 1.0 M $NH_4HCO_3$ are used. The obtained fractions are analyzed by UPLC-MS, and the fraction containing $G_2P_3$ is recovered. The recovered fraction is concentrated and freeze-dried to obtain $G_2P_3$ as a white substance (isolation yield of 20% to 26%).

The HPLC charts of $G_2P_3$, synthesized by the method described above, before and after purification by anion exchange column chromatography are shown in Fig. 1(a) and 1(b), respectively. In the HPLC analysis using absorbance at a wavelength of 260 nm as a detection index, the purity of $G_2P_3$ was confirmed to be 99% or more based on the calculation of the peak area of $G_2P_3$ and the total peak area including other components. The purification conditions are shown below.

Column: BioPro SmartSep Q30 5 mL
Flow rate: 8.0 mL/min
Injection amount: 20 mg/mL, 0.5 mL injection
Eluent A: 0.05 M ammonium bicarbonate aqueous solution, Eluent B: 1.0 M ammonium bicarbonate aqueous solution

$$\text{Gradient (B\%): } 0\% \rightarrow (60\ \text{CV}) \rightarrow 15\% \rightarrow (0.1\ \text{CV}) \rightarrow 100\%\ (5\ \text{CV})$$

[Examples]

[Example 1: Synthesis of $U_2P_4$ using uridine 5'-diphosphate as starting material]

[Formula 10]

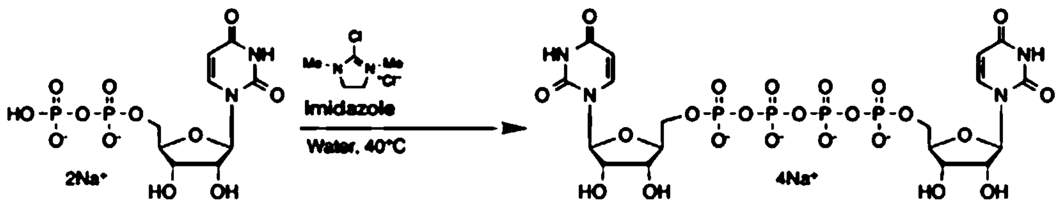


raw material (UDP)
UDP

Diquafosol (U2P4) $U_2P_4$

Uridine 5'-diphosphate disodium salt (20 g, 45 mmol) and imidazole (6.0 g, 90 mmol) were dissolved in deionized water (23 ml), then 2-chloro-1,3-dimethylimidazolinium chloride (7.5 g, 45 mmol) was added, and the mixture was reacted at 40°C for 1 hour. Furthermore, imidazole (6.0 g, 90 mmol) and 2-chloro-1,3-dimethylimidazolinium chloride (7.5 g, 45 mmol) were added and reacted at 40°C for 3 hours. The HPLC chart of the crude product after the reaction is shown in Fig. 2. The reaction solution was adjusted to pH 7 with 1.0 M $NH_4HCO_3$, diluted with deionized water (500 mL), and purified using an ion exchange column (TOYOPEARL GigaCap Q-650M). For elution, solution A: 0.05 M $NH_4HCO_3$ and solution B: 1.0 M $NH_4HCO_3$ were used. The obtained fraction was analyzed by UPLC-MS, and diuridine tetraphosphate ($U_2P_4$) was recovered. The recovered $U_2P_4$ was dissolved in deionized water (100 ml) and passed through Dowex 50Wx4$Na^+$. The eluted solution was concentrated and freeze-dried to obtain $U_2P_4$ tetrasodium salt as a white substance (5.55 g, isolation yield of 30%).

[Example 2: Synthesis of $U_2P_4$ using uridine 5'-monophosphate as starting material]

[Formula 11]

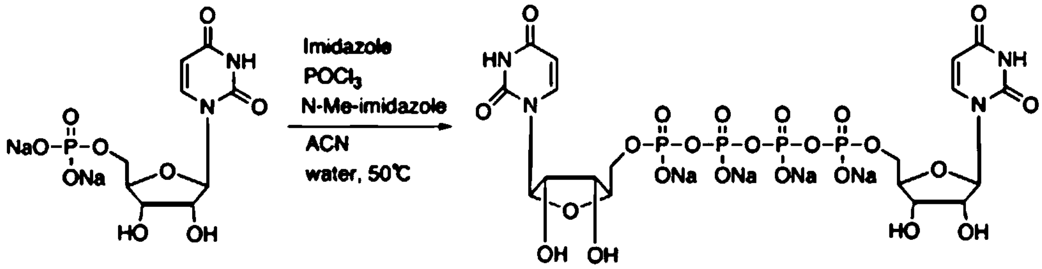


Imidazole (2.45 g, 36 mmol) was dissolved in acetonitrile (24 mL). To the solution, phosphoryl chloride (544 μL, 6 mmol) was slowly added dropwise at room temperature, and the mixture was stirred for 20 minutes, resulting in the precipitation of white crystals. Uridine monophosphate disodium salt (2.21 g, 6 mmol) was added gradually in small portions and the mixture was stirred for 10 minutes. The reaction solution separated into two layers. After the reaction solution was left at 50°C for 10 minutes, 200 μL of deionized water was added dropwise 10 times (a total of 2 mL), and the mixture was stirred for 10 minutes. N-Methylimidazole (1.9 mL, 24 mmol) was added dropwise and reacted at 50°C for 2 to 15 hours. Subsequently, the reaction solution was allowed to return to room temperature, the upper layer was removed by decantation, and a viscous crude product was obtained from the lower layer.

The obtained crude product was diluted with deionized water (50 mL), and purified using an ion exchange column (TOYOPEARL GigaCap Q-650M). For elution, solution A: 0.05 M $NH_4HCO_3$ and solution B: 1.0 M $NH_4HCO_3$ were used. The obtained fraction was analyzed by UPLC-MS, and the fraction containing diuridine tetraphosphate ($U_2P_4$) was recovered. The eluted solution was concentrated and freeze-dried to obtain $U_2P_4$ as a white solid (0.47 g, isolation yield of 17.8%, purity of 98%).

The LCMS spectrum of the liquid extracted from the reaction solution during the reaction is shown in Fig. 2. The MS spectrum of the reaction solution showed peaks corresponding to molecular weights of the starting material uridine monophosphate (UMP), the compound of interest $U_2P_4$, and $U_2P_3$, which is a compound where two uridines are linked with three phosphates, and a substance in which two imidazoles are attached to uridine 5'-diphosphate (hereinafter, the substance is referred to as IntD). One possible structure of IntD is shown below. It was also confirmed by $^{31}$P-NMR measurement that IntD was present in the system (δ7,89 ppm, not shown in the Figure).

[Formula 12]

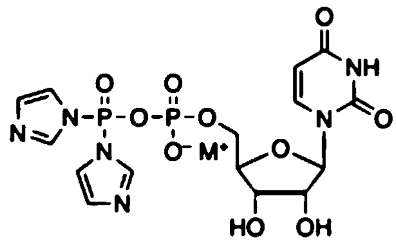

M = imidazolium or sodium

Int D

IntD is an active intermediate, and the starting material is converted to $U_2P_3$ or $U_2P_4$ through this intermediate.

The MS spectrum obtained during the synthesis of $U_2P_4$ is shown in Fig. 3.

Fig. 4 shows the HPLC chart of the crude product obtained after the synthesis reaction in Example 2.

Fig. 5 shows the preparative LC chromatogram of the crude product obtained after the synthesis reaction in Example 2. Fig. 6 shows the HPLC chart of $U_2P_4$ obtained by freeze-drying the Peak 4 fraction solution in Fig. 5.

[Example 3: Synthesis of $G_2P_3$ using guanosine 5'-monophosphate as starting material]

[Formula 13]

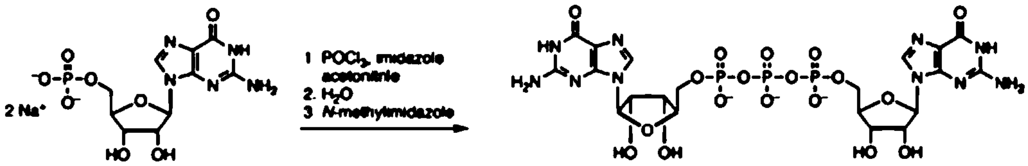


Benzimidazole (18.1 g, 153 mmol) was suspended in acetonitrile (10.3 mL). To the solution, phosphoryl chloride (2.1 mL, 23.5 mmol) was slowly added over 3 minutes at room temperature, and the mixture was stirred for 5 minutes. Then, a previously prepared raw material-catalyst solution in which a guanosine 5'-monophosphate sodium salt (10 g, 24 mmol) and iron(III) chloride (3.8 g, 23.5 mmol) were dissolved in hydrochloric acid at pH 2.0 (23.5 mL) was added dropwise, and the reaction solution was stirred for 30 minutes. Subsequently, the reaction solution was returned to room temperature and adjusted to pH 8.0 with saturated carbonated water, thereby obtaining a crude product solution.

The crude product was diluted with deionized water to 100 mL, and purified using an ion exchange column (YMC Co., BioPro IEX SmartSep Q). For elution, solution A: 0.05 M $NH_4HCO_3$ and solution B: 1.0 M $NH_4HCO_3$ were used. The obtained fractions were analyzed by UPLC-MS, and the fraction containing $G_2P_3$ was recovered. The recovered fraction was analyzed by UPLC-MS, and the fraction containing diguanosine triphosphate ($G_2P_3$) was recovered. The eluted solution was concentrated and freeze-dried to obtain $G_2P_3$ as a white substance (7.1 g, isolation yield of 39.0%, purity of 98%). Fig. 7 shows the HPLC chart of the crude product aqueous solution.

[Example 4: Synthesis of CAP analogue using guanosine derivative as starting material]

[Formula 14]

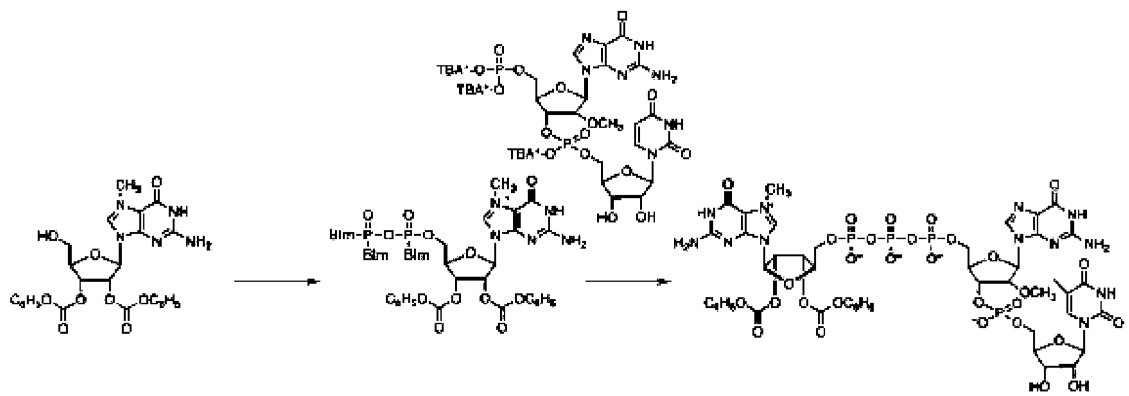

Iron(III) chloride (162 mg, 1.0 mmol) is suspended in acetonitrile (2.0 mL). To the solution, pyrophosphoryl chloride (138 μL, 1.0 mmol) is slowly added dropwise at 0°C, and the mixture is stirred for 20 minutes. 2',3'-Diphenoxyacetyl-N7-methylguanosine (215 mg, 0.4 mmol) is added gradually in small portions, and the mixture is stirred for 10 minutes. A solution of benzimidazole (307 mg, 2.6 mmol) in acetonitrile (0.8 mL) is added dropwise to the reaction solution. After stirring the mixture at room temperature for 30 minutes, a solution of 5'-monophosphorylated rAomeG tetrabutylammonium salt (563 mg, 0.4 mmol) in acetonitrile is added dropwise, and the mixture is stirred for 10 minutes.

Afterwards, the reaction solution was returned to room temperature, and pH was adjusted to 12 using a 1.0 N sodium hydroxide aqueous solution, thereby obtaining a crude product solution.

The crude product solution is diluted with deionized exchange water (10 mL) and purified using an ion exchange column (YMC Co., BioPro IEX SmartSep Q). For elution, solution A: 0.05 M $NH_4HCO_3$ and solution B: 1.0 M $NH_4HCO_3$ are used. The obtained fraction was analyzed by UPLC-MS, and the CAP analogue of interest was recovered. The eluted solution was concentrated and freeze-dried to obtain the target product as a white solid (100 mg, isolation yield of 18.8%, purity of 98%).

[Example 5: Synthesis of CAP analogue using guanosine 5'-monophosphate as starting material]

[Formula 15]

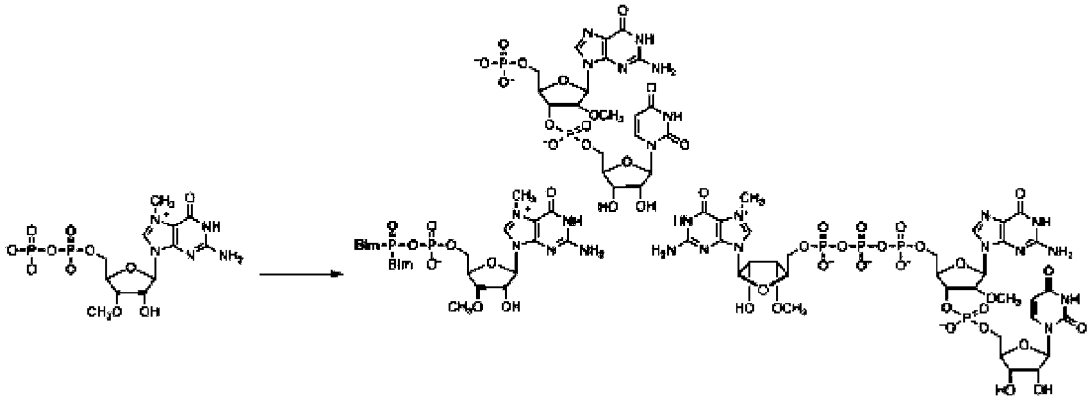

Benzimidazole (307 mg, 2.6 mmol) was suspended in acetonitrile (2.0 mL). To the solution, 2-chloro-1,3-dimethylimidazolinium chloride (203 mg, 1.2 mmol) was slowly added over 3 minutes at room temperature, and the mixture was stirred for 5 minutes. Then, a previously prepared raw material-catalyst solution in which a 5'-diphosphorylated N7-methylguanosine sodium salt (215 mg, 0.4 mmol) and iron(III) chloride (162 mg, 1.0 mmol) were dissolved in hydrochloric acid at pH 3.0 (23.5 mL) was added dropwise at room temperature, and the reaction solution was stirred at room temperature for 30 minutes. Subsequently, a solution of 5'-monophosphorylated rAomeG tetrabutylammonium salt (563 mg, 0.4 mmol) in acetonitrile (10 mL) was added dropwise, and the mixture was stirred for 30 minutes to obtain a crude product solution.

The crude product was diluted with 20% acetonitrile aqueous solution (10 mL) and purified using an ion exchange column (YMC Co., BioPro IEX SmartSep Q). For elution, solution A: 0.05 M $NH_4HCO_3$ and solution B: 1.0 M $NH_4HCO_3$ were used. The obtained fraction was analyzed by UPLC-MS, and the CAP analogue of interest was recovered. The eluted solution was concentrated and freeze-dried to obtain the target product as a white solid (398 mg, isolation yield of 56.0%, purity of 98%). Fig. 8 shows the HPLC chart of the crude product obtained after the reaction.

According to the method for synthesizing the polyphosphorylated nucleoside of the present embodiment, it is possible to produce the polyphosphorylated nucleoside of interest with simple procedures using less expensive raw materials. In addition, it is possible to obtain the target product efficiently through a short process.

According to the 5'-diphosphate-activated nucleotide used in the synthesis of polyphosphorylated nucleoside of the present embodiment, the reaction can proceed using only water or an organic solvent containing water as a solvent. This enables the synthesis method to reduce environmental impact.

**Claims**

**1.** A polyphosphorylated nucleoside having a structure of formula (I):

[Formula 1]

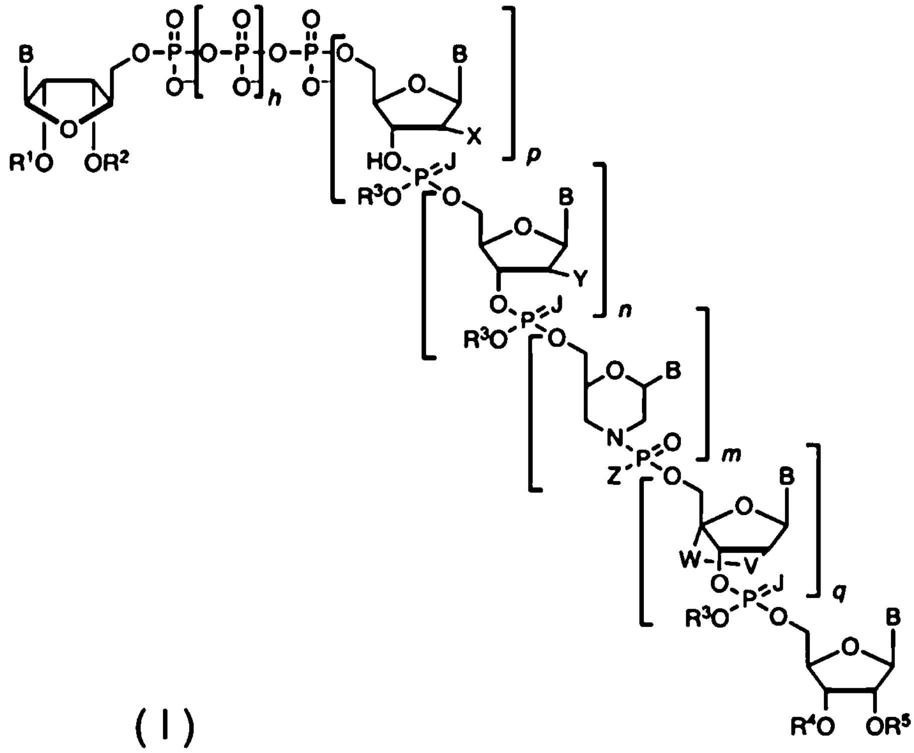

(I)

wherein

B is independently a natural or artificial nucleoside base that is protected by a protecting group or unprotected;
$R^1$ and $R^2$ are each independently H, $CH_2CH=CH_2$, $CH_2C_6H_5$, $CH_2C_6H_4$-p-$OCH_3$, 2-$CH_2C_{10}H_7$, 1-pyrenylmethyl, acetyl, benzoyl, dichloroacetyl, 1-pentenoyl, levulinoyl, phenoxyacetyl, a fatty acid ester having 5 to 22 carbon atoms, an alkyl group having 3 to 11 carbon atoms containing an alkyne moiety, biotin ester, $CONHC_6H_5$, $CONHC_{10}H_7$, Boc, Fmoc, allyloxycarbonyl, benzyloxycarbonyl, TOM, Pivom, CEM, $CH_2OCH_2C_6H_4$-p-$OCH_3$ (PMBOM), or $CH_2O$-2-$CH_2C_{10}H_7$ (NAPOM), and $R^1$ and $R^2$ may be the same or different;
$R^3$ is independently, independently, methyl, ethyl, a vinyl derivative, an allyl derivative, isopropyl, a phenyl derivative, or a polyfluoroalkyl derivative;
$R^4$ and $R^5$ are each independently H, $CH_2CH=CH_2$, $CH_2C_6H_5$, $CH_2C_6H_4$-p-$OCH_3$, 2-$CH_2C_{10}H_7$, 1-pyrenylmethyl, acetyl, benzoyl, dichloroacetyl, 1-pentenoyl, levulinoyl, phenoxyacetyl, $CONHC_6H_5$, $CONHC_{10}H_7$, Boc, Fmoc, allyloxycarbonyl, benzyloxycarbonyl, TOM, Pivom, CEM, $CH_2OCH_2C_6H_4$-p-$OCH_3$ (PMBOM), or $CH_2O$-2-$CH_2C_{10}H_7$ (NAPOM), and $R^4$ and $R^5$ may be the same or different;
X and Y are each independently H, OH, $OCH_3$, $OCH_2CH_2OCH_3$, or F;
J is independently O, S, Se, or BH;
Z is OH, $NH_2$, or $NHCH_3$;
W is $CH_2$, $CH_2CH_2$, or $CH(CH_3)$;
V is O, $NCH_3$, NCOR, or N-N=NH;
h is an integer of 1 or more and 3 or less; and
p, n, m, and q are each 0 or an integer, and p, n, m, and q are in any order, with (p + n + m + q) being an integer of 1 or more and 22 or less.

2. The polyphosphorylated nucleoside according to claim 1, wherein the $R^1$ and $R^2$ are each a dichloroacetyl group, and the $R^3$ is H or a metal ion.

3. The polyphosphorylated nucleoside according to claim 1, wherein the $R^1$ and $R^2$ are each a phenoxyacetyl group, and the $R^3$ is H or a metal ion.

4. A 5'-diphosphate-activated nucleotide used to produce the polyphosphorylated nucleoside having a structure of

formula (I) according to claim 1, the 5'-diphosphate-activated nucleotide having a structure of formula (II):

[Formula 2]

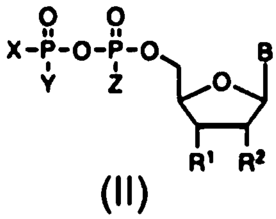

(II)

wherein

B is independently a natural or artificial nucleoside base that is protected by a protecting group or unprotected;
X, Y, and Z are each independently OH, $OR^6$, SH, SR, $BH_3$, halogen, azolide, $NR_2$, or $NH_2$;
$R^1$ and $R^2$ are each independently H, OH, halogen, an alkyl group, a hydroxyl group protected by an acyl-based protecting group, a carbamoyl-based protecting group or a silyl-based protecting group, or a 3 to 11-mer oligonucleotide having a natural or artificial nucleoside base that is unprotected or protected by a protecting group, and $R^1$ and $R^2$ may be the same or different; and
$R^6$ is OBT, OAt, a substituted or unsubstituted vinyl group, or a substituted or unsubstituted allyl group, wherein the substituted vinyl group may contain a cyclic structure.

**5.** A method for synthesizing the 5'-diphosphate-activated nucleotide having a structure of formula (II) according to claim 4, the method comprising steps of:

(a) reacting pyrophosphoryl chloride with a co-catalyst compound in a solvent in the presence or absence of a catalyst compound to prepare a phosphate-activated compound; and
(b) reacting the phosphate-activated compound obtained in step (a) with a nucleoside in which a base moiety is protected by a protecting group or unprotected to prepare a nucleoside 5'-diphosphate chloride, wherein in formula (II), X = Y = Cl, Z = Cl or $O^-$.

**6.** A method for synthesizing the 5'-diphosphate-activated nucleotide having a structure of formula (II) according to claim 4, the method comprising steps of:

(a) reacting a polychlorinating agent with a co-catalyst compound in the presence or absence of a catalyst compound to prepare a phosphate-activated compound; and
(b) reacting the phosphate-activated compound obtained in step (a) with a nucleoside 5'-monophosphate in which a base moiety is protected by a protecting group or unprotected, in a water-hydrophilic organic solvent to prepare a 5'-diphosphate-activated nucleotide, wherein in formula (II), X = Y = azolide or a heterocyclic compound derivative, and Z = azolide, a heterocyclic compound derivative, or $O^-$.

**7.** A method for synthesizing the 5'-diphosphate-activated nucleotide having a structure of formula (II) according to claim 4, the method comprising steps of:

(a) reacting a polychlorinating agent with a nucleoside 5'-diphosphate in which a base moiety is protected by a protecting group or unprotected, in a water-hydrophilic organic solvent in the presence or absence of a catalyst compound to prepare a nucleoside 5'-diphosphate chloride, wherein in formula (II), X = Y = Cl; and
(b) adding a co-catalyst compound to a reaction liquid after step (a) to obtain a 5'-diphosphate-activated nucleotide, wherein in formula (II), X = Y = azolide or a heterocyclic compound derivative, and Z = azolide, a heterocyclic compound derivative, or $O^-$.

**8.** The synthesizing method according to any one of claims 5 to 7, wherein the co-catalyst compound is an imidazole or benzimidazole.

**9.** The synthesizing method according to any one of claims 5 to 7, wherein the catalyst compound is an organic catalyst that is HOBT, HOAu, HOCU, or DMT-MM, iron(III) chloride, aluminum chloride, zinc chloride, copper(II) chloride,

magnesium chloride, cesium chloride, cerium triflate, or scandium triflate.

**10.** The synthesizing method according to claim 6 or 7, wherein the polychlorinating agent is phosphoryl chloride, dichlorophosphoric anhydride, thionyl chloride, or 2-chloro-1,3-dimethylimidazolinium chloride.

**11.** A method for synthesizing the polyphosphorylated nucleoside having a structure of formula (I) according to claim 1, the method comprising a step of:
condensing the 5'-diphosphate-activated nucleotide described in any one of claims 5 to 7 with a nucleoside 5'-phosphate in the presence of water to obtain a polyphosphorylated nucleoside.

**12.** The synthesizing method according to claim 11, wherein the nucleoside 5'-phosphate is a 5'-monophosphorylated nucleic acid selected from guanosine 5'-phosphate, N7-methylguanosine 5'-phosphate, RNA, DNA, a DNA/RNA chimera, or a modified nucleoside-containing RNA.

FIG. 1

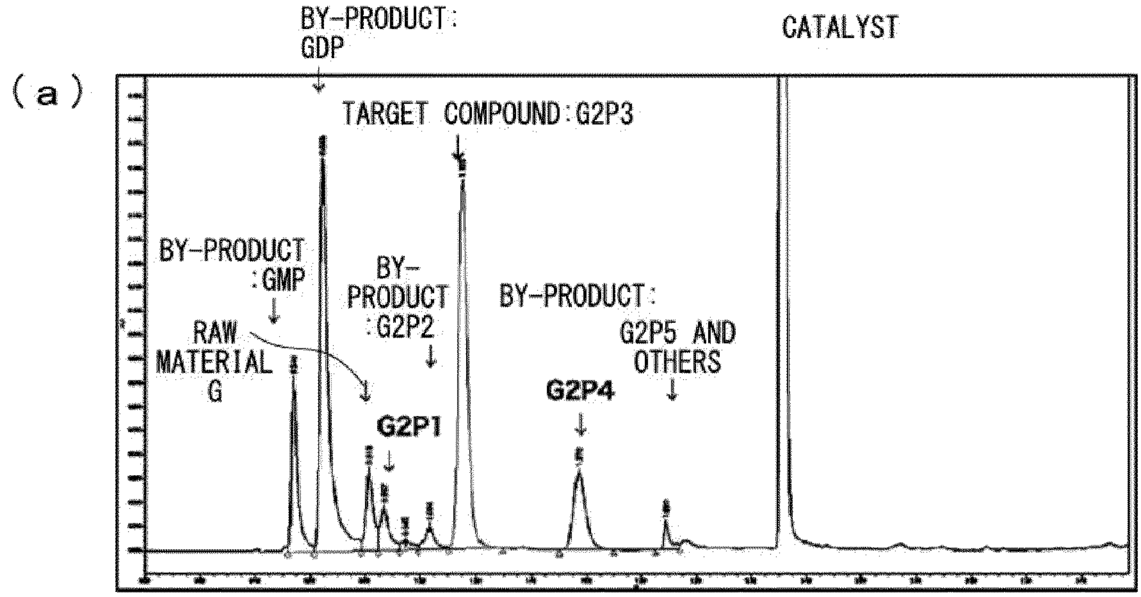
(a)
BY-PRODUCT: GDP
CATALYST
TARGET COMPOUND:G2P3
BY-PRODUCT :GMP
RAW MATERIAL G
BY-PRODUCT :G2P2
BY-PRODUCT:
G2P5 AND OTHERS
G2P4
G2P1

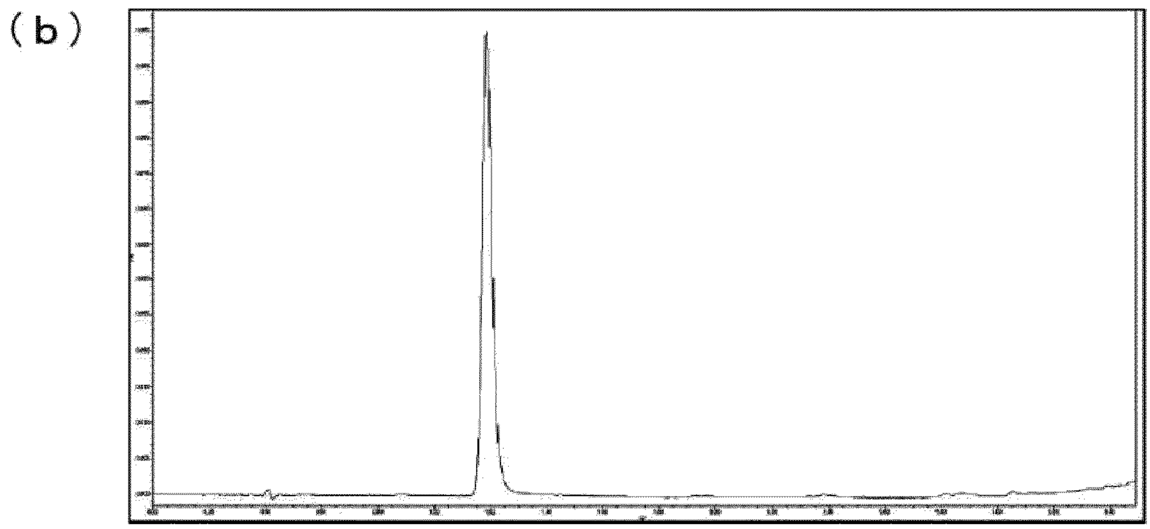
(b)

FIG. 2

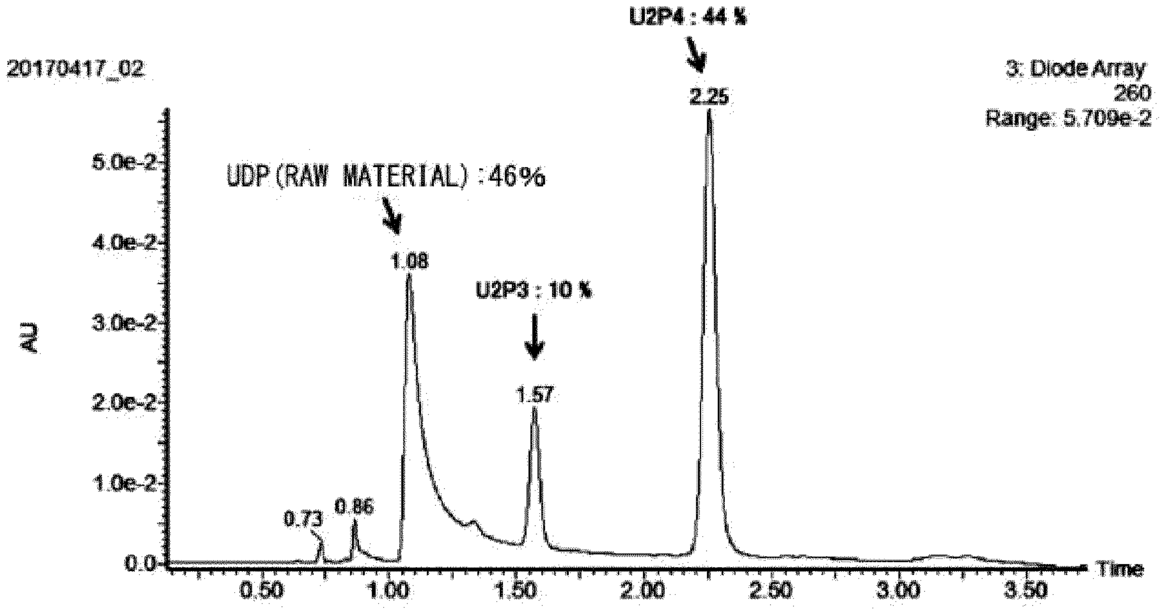
U2P4 : 44 %
20170417_02
3: Diode Array
260
Range: 5.709e-2
2.25
5.0e-2
UDP(RAW MATERIAL):46%
4.0e-2
1.08
U2P3 : 10 %
3.0e-2
AU
2.0e-2
1.57
1.0e-2
0.73
0.86
0.0
0.50
1.00
1.50
2.00
2.50
3.00
3.50
Time

FIG. 3

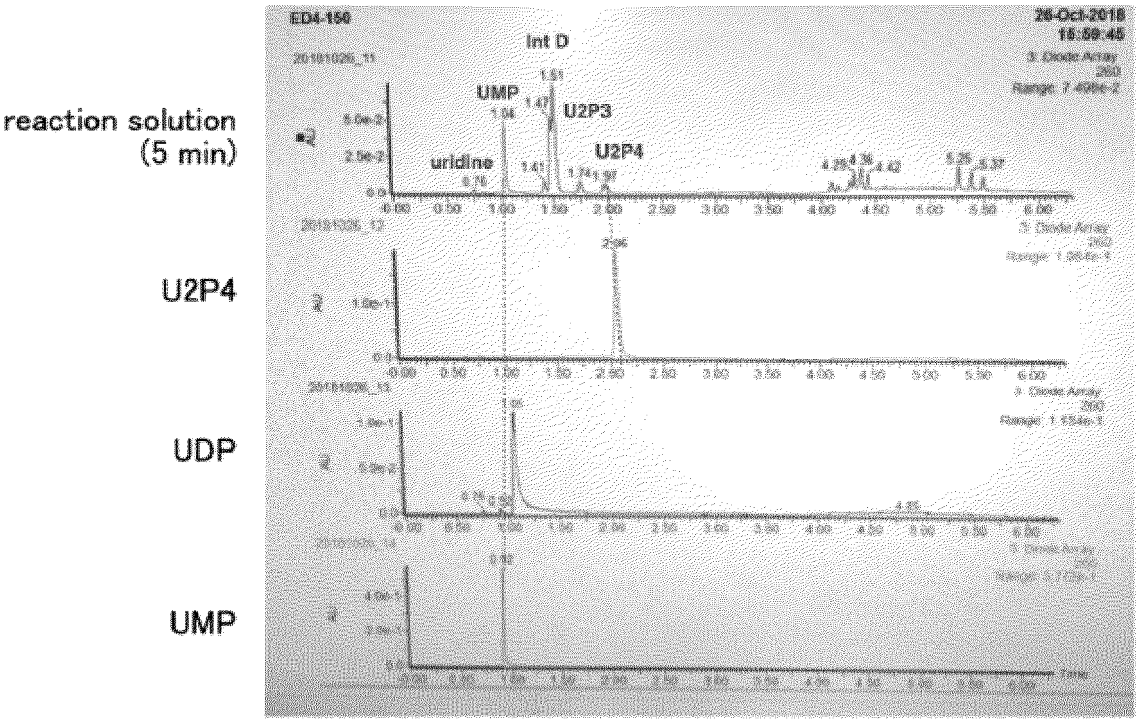
reaction solution
(5 min)
U2P4
UDP
UMP
ED4-150
26-Oct-2018
15:59:45
Int D
UMP
U2P3
U2P4
uridine

FIG. 4

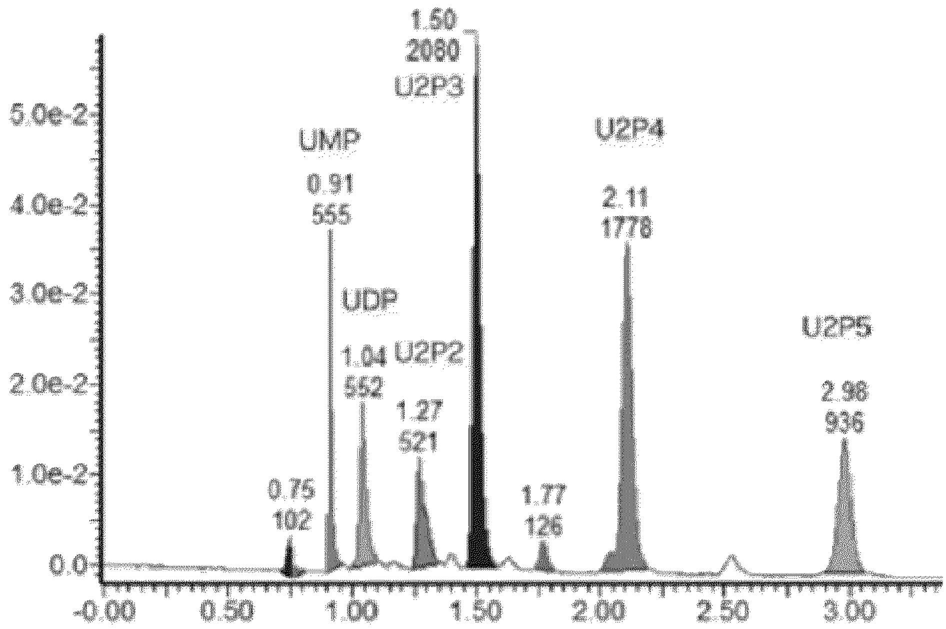
1.50
2080
U2P3
UMP
0.91
555
U2P4
2.11
1778
UDP
1.04
552
U2P2
1.27
521
U2P5
2.98
936
0.75
102
1.77
126
5.0e-2
4.0e-2
3.0e-2
2.0e-2
1.0e-2
0.0
-0.00
0.50
1.00
1.50
2.00
2.50
3.00

FIG. 5

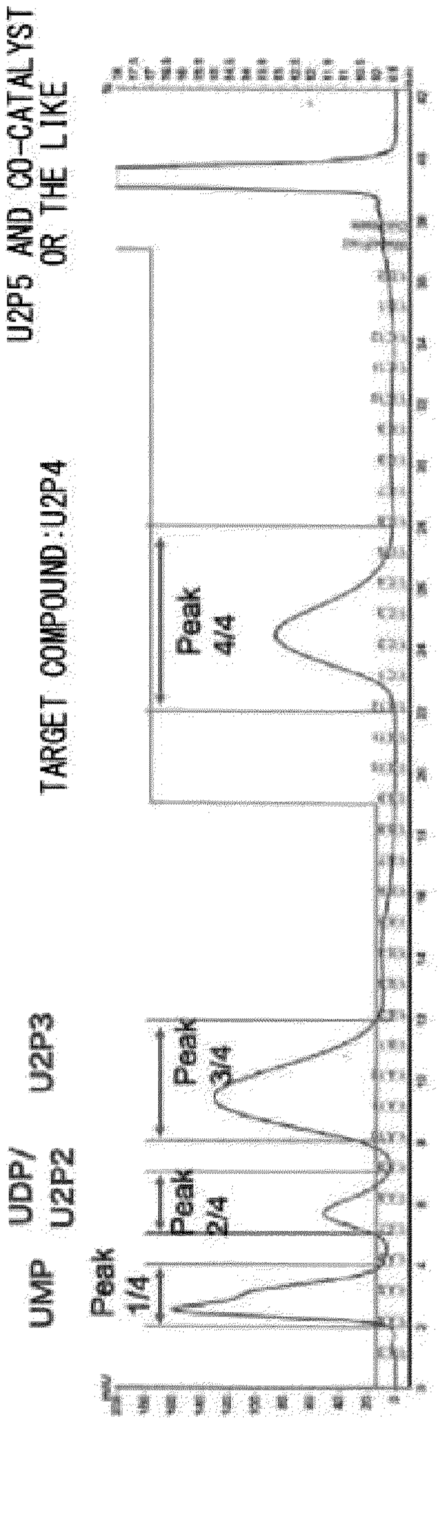
U2P5 AND CO-CATALYST OR THE LIKE
TARGET COMPOUND:U2P4
Peak 4/4
U2P3
Peak 3/4
UDP/ U2P2
Peak 2/4
UMP
Peak 1/4

FIG. 6

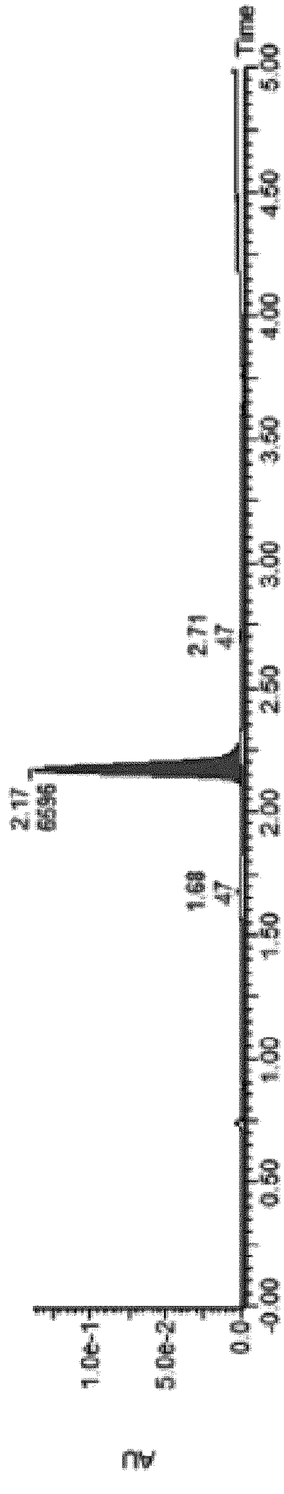

2.17
6596
1.68
47
2.71
47
AU
1.0e-1
5.0e-2
0.0
-0.00
0.50
1.00
1.50
2.00
2.50
3.00
3.50
4.00
4.50
5.00
Time

FIG. 7

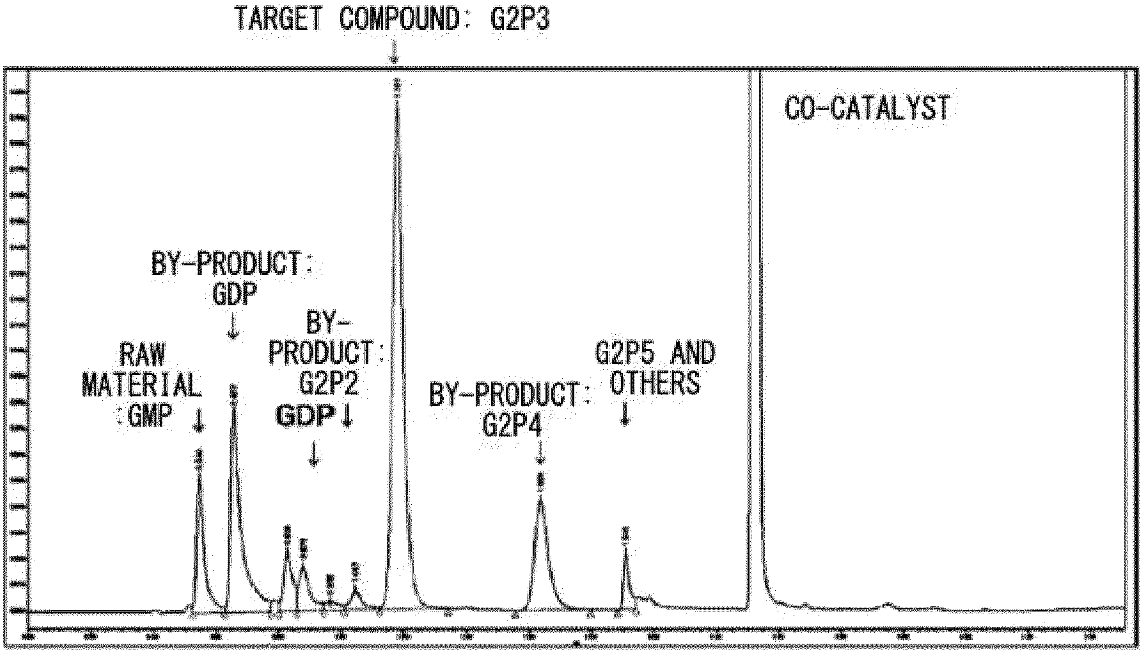
TARGET COMPOUND: G2P3
CO-CATALYST
BY-PRODUCT: GDP
BY-PRODUCT: G2P2
GDP
RAW MATERIAL: GMP
BY-PRODUCT: G2P4
G2P5 AND OTHERS

FIG. 8

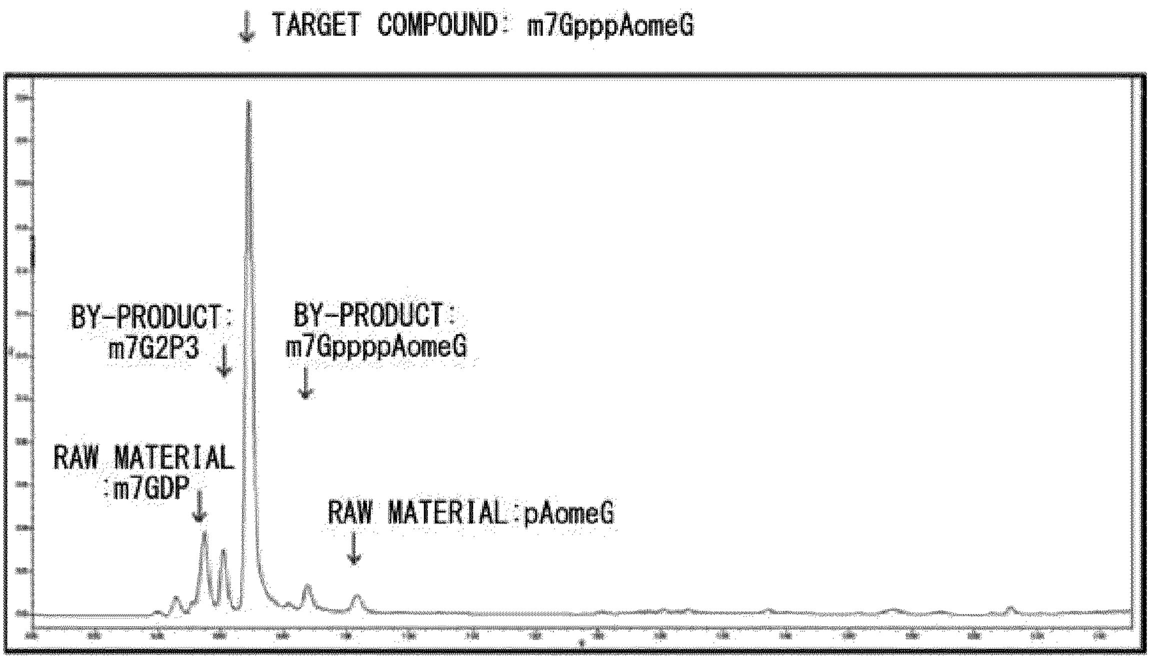
↓ TARGET COMPOUND: m7GpppAomeG
BY-PRODUCT:
m7G2P3
BY-PRODUCT:
m7GppppAomeG
RAW MATERIAL
:m7GDP
RAW MATERIAL:pAomeG

## INTERNATIONAL SEARCH REPORT

International application No.
**PCT/JP2024/007991**

**A. CLASSIFICATION OF SUBJECT MATTER**

***C07H 19/20***(2006.01)i; ***C07H 21/02***(2006.01)i
FI: C07H19/20; C07H21/02

According to International Patent Classification (IPC) or to both national classification and IPC

**B. FIELDS SEARCHED**

Minimum documentation searched (classification system followed by classification symbols)

C07H19/20; C07H21/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2019/036682 A1 (MODERNATX, INC.) 21 February 2019 (2019-02-21)<br>claims, examples | 1-3 |
| Y | | 11-12 |
| A | | 4-10 |
| X | CN 113603739 A (HONGENE BIOTECHNOLOGY LIMITED) 05 November 2021 (2021-11-05)<br>claims, examples | 1-3 |
| Y | | 11-12 |
| A | | 4-10 |
| X | CN 114941018 A (YEASEN BIOLOGICAL TECHNOLOGY (SHANGHAI) CO., LTD.) 26 August 2022 (2022-08-26)<br>claims, examples | 1-3 |
| Y | | 11-12 |
| A | | 4-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 May 2024** | **21 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/007991**

**C. DOCUMENTS CONSIDERED TO BE RELEVANT**

| Category* | Citation of document, with indication, where appropriate, of the relevant passages | Relevant to claim No. |
|---|---|---|
| X | CN 113957108 A (HONGENE BIOTECH CORPORATION) 21 January 2022 (2022-01-21) claims, examples | 1-3 |
| Y | | 11-12 |
| A | | 4-10 |
| X | US 2003/0194759 A1 (DARZYNKIEWIZ, Edward) 16 October 2003 (2003-10-16) claims, examples, fig. 1 | 4 |
| Y | | 11-12 |
| A | | 1-3, 5-10 |
| X | WO 2008/016473 A2 (APPLERA CORPORATION) 07 February 2008 (2008-02-07) claims, examples | 4 |
| Y | | 11-12 |
| A | | 1-3, 5-10 |
| X | US 2004/0014713 A1 (YERXA, Benjamin R.) 22 January 2004 (2004-01-22) paragraph [0039] | 4 |
| A | | 1-3, 5-12 |

Form PCT/ISA/210 (second sheet) (July 2022)

**INTERNATIONAL SEARCH REPORT**
**Information on patent family members**

International application No.
**PCT/JP2024/007991**

| Patent document cited in search report | Publication date (day/month/year) | Patent family member(s) | Publication date (day/month/year) |
|---|---|---|---|
| WO 2019/036682 A1 | 21 February 2019 | JP 2020-532963 A<br>US 2019/0309337 A1<br>EP 3668971 A1<br>CN 111212905 A | |
| CN 113603739 A | 05 November 2021 | WO 2023/025073 A1 | |
| CN 114941018 A | 26 August 2022 | (Family: none) | |
| CN 113957108 A | 21 January 2022 | (Family: none) | |
| US 2003/0194759 A1 | 16 October 2003 | (Family: none) | |
| WO 2008/016473 A2 | 07 February 2008 | JP 2009-544754 A<br>US 2010/0261231 A1<br>EP 2049665 A1 | |
| US 2004/0014713 A1 | 22 January 2004 | JP 2001-510484 A<br>WO 1999/005155 A2<br>EP 1012154 A2<br>CN 1265114 A<br>KR 10-2001-0013797 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008012949 A
- WO 2014103704 A
- WO 2017053297 A
- WO 2022212442 A